# EUROPEAN PATENT APPLICATION

(11) **EP 2 103 313 A1**
(43) Date of publication of application: **23.09.2009**
(21) Application number: 08152979.4
(22) Date of filing: 19.03.2008
(51) Int. Cl.: A61K 49/22, A61K 41/00, A61K 9/51, B01J 13/12

(54) **Method for the synthesis of hollow spheres**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to a method of synthesizing acoustically active biodegradable hollow spheres. By using water soluble organic solvents and pegylated polymers, direct precipitation of these spheres is facilitated leading to a fast and convenient preparation route.

## Description

### FIELD OF THE INVENTION

The present invention relates to a method for the synthesis of hollow spheres with a polymeric shell. Moreover, the invention relates to hollow spheres and the use of these spheres in ultrasound imaging or ultrasound drug delivery.

### BACKGROUND OF THE INVENTION

Ultrasound is used in clinical settings as a diagnostic imaging technique. It is relatively inexpensive and more important, does not use ionizing radiation. In ultrasound imaging, sound waves are transmitted via a transducer into biological tissue. Depending on the acoustic properties of the underlying tissue and fluids, the sound waves can be fully or partially reflected or absorbed. Reflected sound waves are detected by a receiving transducer and are further processed to form an image of the tissue under investigation. The contrast of the image is determined by the relative differences in acoustic properties of the tissues. Although ultrasound technology is advancing, imaging and detection of malignancies and disease in main organs such as liver, spleen, kidney, prostate and the vasculature is still technically challenging.

In order to optimize the image quality that can be obtained in ultrasound imaging, contrast agents have been developed. Existing ultrasound contrast agents are designed to be acoustically active and comprise composition such as gas-filled micro bubbles stabilized with a lipid, polymer or protein shell. Acoustic properties, particularly acoustic impedance of solids, liquids and gases are different. These distinct changes in acoustic impedance and compressibility result in a more intense reflection of sound waves at interfaces between solids, liquids and gases, leading to an increase in intensity in the ultrasound image. Gas-filled ultrasound contrast agents also react to the ultrasound waves by compression and expansion, giving rise to the generation specific resonance frequencies that can be detected selectively. Additionally, contrast agents can be activated with an ultrasound pulse in the sense that they can rupture releasing detectable gas, and, in case drugs are included in the agent, locally releasing a therapeutic agent. Other modes for delivery include alteration of the surrounding tissue by the interaction of the newly released gas bubble, and also temporary increase in permeability of the vessel walls to drug incorporated or administered separately from the contrast agent.

Hollow polymer-shelled agents can be synthesized from emulsification procedures where an oil filled, polymer coated capsule is created followed by removal of the core with a freeze-drying process. An example of such a synthesis route is disclosed in an article by Le et al. (IEEE, Issue, 22-23 March 2003 Page(s): 315 - 316) which discloses the synthesis of nanocapsules through the adaptation of an emulsion polymer microcapsule fabrication method. To make oil-filled capsules an oil or other hydrophobic additive is added to a polymer solution in a good solvent for the polymer such as dichloromethane. This solution is subsequently emulsified in an aqueous phase containing a stabilizer. The good solvent is removed during processing. As a result, the solubility of the polymer in the emulsion droplet will decrease and subsequently phase separate from the oil-rich phase, thus forming a polymer-shelled oil containing capsule. To make a hollow core via an emulsification method, the encapsulated hydrophobic additive or oil is removed by freeze-drying. Stabilizers such as poly vinyl alcohol (PVA) are normally added to the aqueous phase to control particle size and to prevent aggregation. Addition of stabilizers often has to be done in an excess amount and thus requires subsequent purification and or washing steps leading to reduced process yield.

### SUMMARY OF THE INVENTION

It is an object of the present invention to improve the known method for the synthesis of hollow spheres with acoustic properties in order to obtain a fast and convenient preparation.

According to the invention, this object is realized by the method comprising the following steps:
(a) mixing a solution comprising a polymer (1) with a hydrophilic block, a hydrophobic compound, a water miscible organic solvent with an aqueous solution
(b) removal of water miscible organic solvent
(c) lyophilizing to remove hydrophobic alkane

Introduction of a hydrophilic block on polymers that will form the shell of the spheres facilitates stabilization without using additives. An example of such a hydrophilic block is a large hydrophilic group such as polyethlylene-glycol (PEG). Direct precipitation of spheres is induced directly by the mixing instead of by slow solvent removal as the organic solvent will immediately distribute homogeneously in the aqueous phase. The method according to the invention is faster and more convenient then conventional emulsification procedures. An additional feature of the use of polymers with a hydrophilic block is the increased circulation time of the particles relative to particles without these blocks. Advantageously, the synthesis of spheres with a size below 1 micron is facilitated.

According to another embodiment, the hydrophilic organic solvent is selected from the group comprising acetone, tetrahydrofuran, dimethylsulfoxide, dimethylformamide, dimethylacetamide or a combination thereof. These solvents are good solvents for polymers used in the method according to the invention and mix with water. Removal of the solvent can be conveniently facilitated by evaporation.

In a preferred embodiment, the polymer (1) is selected from the group comprising polylactide, polycaprolactone, polycyanoacrylate and copolymers of one of the foregoing, copolymers of polyglycolide, or any combination thereof. These polymers are polymers that, when used in the method according to the invention, will lead to the formation of a sufficiently rigid polymer shell. Biodegradability is desired when hollow spheres are used in human and medical applications such as ultrasound imaging.

According to a preferred embodiment, an additional polymer (2) comprises an alkyl or a fluorinated end group or a combination thereof. The introduction of alkyl and or fluor groups on the polymer results in the generation of a hydrophobic inside of the hollow sphere, ensuring water repellent properties. This will stabilize the spheres in an aqueous biological environment. Stable acoustically active spheres can be obtained by using alkyl groups with 8 to 14 carbon atoms for a polymer molecular weight around 3000-5000.

Use of different combinations of polymers, as well as different modification levels of the polymers can be envisioned. Polymers with a hydrophilic block can be combined with hydrophobically modified polymers.

Another embodiment of the method according to the invention is that the solution of step (a) additionally comprises a hydrophobic compound such as an oil that remains after lyophilization and a pharmaceutical or diagnostic compound. A preferred example of such a hydrophobic compound is at least one of a higher alkane, lipid, parafin or oil, more preferably hexadecane. This addition facilitates the creation of a biodegradable hollow sphere that contains, next to a gas, a drug dissolved or finely dispersed in the remaining hydrophobic compound. It is possible to release locally the drug dissolved in the compound that remains inside the biodegradable hollow sphere by using an ultrasound pulse.

Another embodiment of the invention is a hollow sphere with a polymeric shell synthesized according to a method comprising the following steps:
(a) mixing a solution comprising a polymer with a hydrophilic block, a hydrophobic compound, a water miscible organic solvent with water
(b) removal of the water miscible organic solvent
(c) lyophilizing to remove hydrophobic alkane

This hollow sphere preferably is able to rupture upon application of diagnostic or therapeutic ultrasound. Hollow spheres according to this embodiment of the invention are able to rupture when an ultrasound pulse is applied. This behavior leads the formation of free gas bubbles that can interact with ultrasound in the area targeted by the ultrasound pulse, enhancing the ultrasound guidance for the drug delivery. In a similar way a pharmaceutical or diagnostic compound can also be released in the same area. This opens the possibility to control the amount and the location of delivery. Furthermore, the newly-formed gas bubbles readily absorb incident ultrasound energy and then re-radiate as secondary acoustic sources, increasing local ultrasound energy absorption. In turn, the locally increased ultrasound energy deposition may increase the stimulation of local tissue immune response and enhance the sono-poration and thus the drug uptake. Also, the local ultrasound energy absorption can be controlled by the amount of encapsulated gas (i.e., amount of lyophilizable compound) and the activation amplitude of the acoustic pulses. The appearance of a unique acoustic signature corresponding to gas bubbles of a specific size allows for identification of activated release as well as ideally allowing some degree of confidence in the concentration of drug released.

The hollow spheres preferably have a size below 1 micron to increase the circulation time by avoidance or rate suppression of the primary clearance mechanisms in the body.

In another embodiment of the invention, the hollow sphere comprises a gas precursor. According to this embodiment, the sphere can contain liquid perfluorocarbons that can phase convert, like perfluorohexane, perfluorheptane, perfluoroctane and perfluoroctylbromide. Compared to gas filled hollow spheres, the spheres according to this embodiment of the invention have longer circulation times. Furthermore, as gas formation is only induced by the application of ultrasound, the risk of forming uncontrollable large gas bubbles is minimized.

In another embodiment, the shell of the hollow sphere comprises a targeting moiety. By coupling a targeting moiety or pre-targeting moiety to the sphere, selective targeting of the sphere is facilitated. Examples of targeting moieties include but are not limited to antibodies or antibody fragments, biotin/streptavidin linkers and chemical orthogonal coupling moieties such as phosphine and azide groups for Staudinger reactions.

In another embodiment, the hollow sphere comprises a pharmaceutically active compound so the sphere can function as a drug carrier. Among others, the pharmaceutically active compound can be selected from the group comprising antibodies, proteins, siRNA, shRNA and pDNA.

In another embodiment according to the invention, the hollow sphere comprises an imaging agent selected from the group comprising PET, SPECT or MRI agents. By combining the ultrasound characteristics of the hollow sphere with an additional imaging moiety, it is facilitated to follow eg. degradation routes of the polymeric shell or local drug delivery.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiments described hereinafter.

### BRIEF DESCRIPTION OF THE DRAWINGS

Fig. 1: Schematic representation of polymer
Fig. 2: Activation of spheres synthesized according to the invention.

### DETAILED DESCRIPTION OF THE EMBODIMENTS

It should further be noted that product names and names of the suppliers used in the tables and examples might be subject to trademark rights and are not intended to be used in a generic way but only to define the specific products supplied by the named suppliers.

The invention will be illustrated with reference to the following, non-limiting examples, preferred embodiments and non-limiting figures.

The method according to the invention provides a fast and efficient synthesis route for the generation of acoustically active hollow spheres. Acoustically active means that these particles can rupture upon the application of ultrasound of 1MHz above a threshold of at most 1 MPa and an intensity of for example 2 W/cm². Sphere indicates a particle or moiety with a spherical or approximately spherical shape including but not limited to oval and/or partially compressed spheres.

### Method according to the invention

Acoustically active hollow spheres can be created by using emulsification techniques. Emulsification relies on solvents that are immiscible with water or, to a very limited extend, miscible such as halogenated solvent for instance dichloromethane. Generally, additives are needed to control the size of the particles that are formed and to prevent the aggregation of particles during freeze drying. These methods generally result in hollow spheres with a size in the micron range.
In the present invention we improve the known method for the synthesis of hollow spheres with acoustic properties in order to obtain a fast and convenient preparation, especially suitable for the preparation of submicron hollow spheres.
The steps that can be identified in the synthesis method according to the invention are described in detail below.

### Step (a)

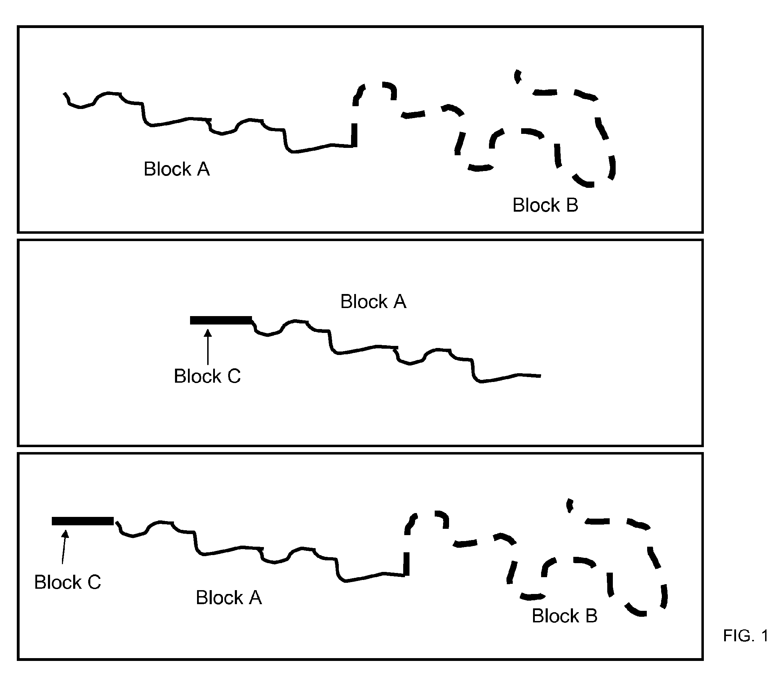

In step (a) of the method according to the invention, a mixture is provided from a solution that comprises a polymer with a hydrophilic block, a hydrophobic compound like and alkane or oil, a hydrophilic organic solvent which is added to water.
A polymer or a combination of polymers is used to form the shell of the hollow spheres. The polymers used for the method according to the invention can comprise different elements as schematically shown in figure 1.

A polymer used in the method according to the invention preferably comprises synthetic- or biopolymer blocks elements that largely determine the mechanical properties of the rigid (for example crystalline or glassy) shell. Typical biodegradable polymers that fulfill these requirements are polylactide (PLA), in either L or DL form or copolymers thereof. This or these elements form the backbone of the polymer that predominate in the shell formation of the hollow particle and are schematically represented by block (A) in figure 1. Relatively low molecular weight polymers have less entanglement in the shell and will therefore more easily lead to shell rupture upon the application of ultrasound. Provided that the mechanical stability is sufficient molecular weight of less than 10,000, more preferably 2,000 to 10,000 are preferred.

A mixture of polymers with different modifications can be used and in figure 1, 3 examples are shown. According to the invention, a polymer with a hydrophilic block is present in the mixture. This preferably bulky hydrophilic group (schematically represented by block B) will be located on the outside of the sphere that is created by the method according to the invention. An example of such a group is polyethyleneglycol. Polymers containing a poly-ethylene oxide block can be incorporated to at least 30% of the total polymer weight.

The polymer preferably comprises hydrophobic moieties. Examples of such a moiety are alkyl groups or fluorinated groups. These groups are schematically represented in figure 1 by block C. Without wishing to be bound by any theory it is believed that in the preparation process these groups will orient towards the core side of the capsule, providing a hydrophobic interior. By using these hydrophobic blocks, stability of the hollow spheres in a biological environment is enhanced due to water repellent properties.

The use of a combination of a polymer with a hydrophilic block and a hydrophobically modified polymer is preferred in the method according to the invention. Use of a polymer that comprises both a hydrophilic group and hydrophobic moieties such as shown in the lower panel in figure 1 can also be envisioned.

Instead of producing an emulsion using a solvent that hardly mixes with the aqueous solution, we use a hydrophilic organic solvent that mixes completely with water. Examples of such a solvent are acetone, tetrahydrofurane, dimethylsulfoxide, dimethylformamide and dimethylacetamide. As these solvent are good solvents for the polymer as well, removal of the hydrophilic solvent leads to precipitation of the polymer on the droplets of hydrophobic solvent in water.

The hydrophobic compound is included as a bad solvent for the shell-forming polymer. The choice of such a substance should be made such that it can be removed with freeze-drying. Therefore the compound to be removed should be able to become solid and have a reasonable vapor pressure under freeze drying conditions so it can be removed. Cyclo-octane and cyclodecane are suitable examples.

To create an emulsion of the mixture, preferably stirring or another form of agitation/shear forces is applied. Optionally further emulsification treatment is included to form an emulsion with the desired, preferably monodispersed, particle size distribution. Suitable equipment to obtain such emulsification treatment is for example selected from colloid mills, homogenizers, sonicaters. Optionally the emulsion either before or after such treatments, is pressed through a glass filter. When desired such treatment may be repeated multiple times.

### Step (b)

In step (b) of the method according to the invention, the hydrophilic solvent is removed, for example at reduced pressure. Another suitable way to remove the hydrophilic solvent is to increase the temperature for example to a temperature from 25 to 35 °C, or simply by stirring the mixture for a given amount of time. Without wishing to be bound by any theory it is believed that whilst the hydrophilic solvent vaporizes the concentration of the shell composition in the emulsion internal phase increases to over the solubility threshold and at such moment in time the shell composition will start to precipitate.

This precipitation then leads to the formation of a shell of polymer at the surface of the emulsion inner phase (emulsion droplet) comprising the hydrophobic solvent. It is believed that once the majority or all of the hydrophilic solvent has vaporized, a shell composition results which covers a core comprising non-solvent and optionally other ingredients that may have been added at an earlier stage of the process.

### Step (c)

By using a polymer with a hydrophilic block, the addition of a stabilizer is no longer needed, leading to a sample from step (b) that can be freeze-dried directly without the use of any purification steps. By freeze drying, the hydrophobic compound that constitutes the core of the sphere is removed, leading to a gas filled or partially gas filled spherical particle. In addition it is preferred that this non-solvent for the polymer sublimes rather than evaporates. Therefore a non-solvent with a melting point not far below 0°C is preferred. Other non-solvents that can be removed in a freeze-drying process may also be considered where it is believed to be preferred but not always absolutely necessary that the non-solvent is solid in the freeze-drying process. For example, alkanes or cycloalkanes with 8 to 14 carbohydrate moieties can be used.

### Hollow sphere according to the invention

Until now, an efficient synthesis route for ultrasound contrast agents with a size in below the micron range was absent. Reduction of size is thought to lead to a drastic decrease of the contrast at the frequencies commonly employed in echography as the resonance frequency is a distinct function of the size of the contrast agent. On the other hand, the preparation of very small hollow spheres is troublesome as the Laplace pressure dictates that small bubbles are unstable and Ostwald ripening of a hollow spheres preparation will lead to the loss of gas from small bubbles and growth of larger hollow spheres. Therefore stable nanometer sized hollow sphere preparations are scarce which has hampered the development of the understanding of their acoustic behavior. It should be noted that the absence of background signal from nano-spheres in circulation may be of advantage in some drug delivery protocols where only the activated nano-spheres (those whose gas has been freed from the polymer shell) are desirable for detection.

The method according to the invention facilitates the synthesis of stable hollow spheres with a size below the micron range.

Ultrasound contrast agents are investigated for many new applications such as molecular imaging, drug delivery and the combination thereof. For these applications long circulation times, on the order of hours, are required to obtain efficient accumulation at the region of interest which is the case for targeted contrast agents for other modalities (MRI agents) and drug delivery vehicles. An increased circulation time can be established by reducing the size of the agent to well below 1 um. Furthermore, steric hydrophilic blocks such as PEG increase circulation times by decreasing the uptake of the spheres via the RES.

Drug delivery vehicles can be prepared having a drug or contrast agent directly associated to the sphere. These drugs or contrast agent can be incorporated in the shell, attached to the shell as drug-carrying particles such as liposomes or encapsulated in the core (inside the shell). If the drug is incorporated in the core, a preferred option is to have them dissolved in a solvent that cannot be freeze-dried. Examples of such solvents are for instance hexadecane or lipids such as triglycerides such as tricaprylin. Drugs to be included in such a system are preferably hydrophobic drugs, paclitaxel being a common example. For hydrophilic drugs the option of attaching a liposome to the outer shell, the liposome containing the hydrophilic drug, is most preferred.

Another possible approach is to allow for the re-suspension of lyophilized nano-spheres and then combination either within the vial or through adjoining administration techniques (different syringe pumps tied to the same venous or arterial access) with a desired drug or biologic. This "proximity" delivery technique makes use of the increase in permeability of the vessel walls and localized cells for the co-administered drug or biologic.

Next to the incorporation of drugs, specific targeting of nanobubbles is also possible. This can for example be facilitated via biotin streptavidin coupling agents. Biotinylated pla-peo can be synthesized to which streptavidin can be added followed by attachment of a biotinylated antibody or fragment thereof. Direct coupling is also a possibility for instance using thio-esters. Other targeting agents could be but are not limited to antibody fragments or chemical orthogonal reaction couples such as phosphine and azide groups to facilitate Staudinger ligation or reaction.

The current invention comprises a method to conveniently create gas-filled spheres, from a polymer solution in a non-halogenated organic solvent without added stabilizer by a nano-precipitation process creating hydrophobic solvent filled polymer-shelled capsules. This process is directly followed by removal of the hydrophobic solvent using a freeze-drying process.

It is to be understood that although preferred embodiments, specific mixtures and materials have been discussed herein for the method and hollow spheres according to the present invention, various changes, modifications or combinations in form and detail may be made without departing from the scope and spirit of the invention.

### EXAMPLE

### Synthesis

A solution of hydrophobically modified PLLA (L-polyactide) in aceton with an average molecular weight of between 2000 and 5000 was mixed with a block copolymer of PLLA and PEO of molecular weight ratio 5000:700 or a block copolymer of PLLA and PEO (polyethylene oxide) of a molecular weight ratio of 1800:2000. Cyclodecane was added to this solution and the solution was thoroughly mixed with water and pressed through a 1 µm filter. The acetone was removed by evaporation in a rotating flask under reduced pressure. Poly-ethlyene glycol was added as a freeze-drying additive and freeze-drying took place as described elsewhere. Dynamic light scattering measurements showed a hydrodynamic diameter of 200 nm after freeze-drying. The nanobubbles were observed to float to the top of a tube upon centrifugation, differential scanning calorimetry showed that the cyclodecane had been removed. As the particles float to the top this proves that their density is below 1 g/cm³ and as the density of the polymer is about 1.25 g/cm³ this proves that gas is associated with the constructs.

The tables demonstrate a number of compositions that have been made including the average bubble sizes measured by dynamic light scattering before and after freeze-drying, including the polydispersity index, all these compositions showed floating particles after re-suspension indicating the inclusion of gas:

| **Polymer blend** | **Copolymer ratio** | **Polymer: cyclodecane ratio** | **Size after filtr. and solvent evap.** | **PI** | **Size after freeze drying** | **PI** |
|---|---|---|---|---|---|---|
| mPEG(**700**)-PLA(**5000**)/PLA-PFO | 1:9 | 1:1 | 180nm | 0.14 | 215nm | 0.2 |
| mPEG(**700**)-PLA(**5000**)**/**PLA-PFO | 1:9 | 1:3 | 300nm | 1.69 | 280nm | 0.2 |
| mPEG(**700**)-PLA(**5000**)/PLA-PFO | 1:9 | 1:5 | 232nm | 1.02 | 290nm | 0.1 |
| mPEG(**700**)-PLA(**5000**)/PLA-PFO | 2:8 | 1:1 | 185nm | 0.18 | 210nm | 0.2 |
| mPEG(**700**)-PLA(**5000**)/PLA-PFO | 2:8 | 1:3 | 300nm | 0.06 | 225nm | 0.1 |
| mPEG(**700**)-PLA(**5000**)/PLA-PFO | 2:8 | 1:5 | 460nm | 0.62 | 240nm | 0.2 |

| **Polymer blend** | **Copolymer ratio** | **Polymer: cyclodecane ratio** | **Size after filtr. and solvent evap.** | **PI** | **Size after freeze drying** | **PI** |
|---|---|---|---|---|---|---|
| mPEG(**2000**)PLA(**1800**) /PLA-PFO | 1:9 | 1:1 | 160nm | 0.03 | Not measured | * |
| mPEG(**2000**)PLA(**1800**) /PLA-PFO | 1:9 | 1:3 | 160nm | 0.11 | 200nm | 0.2 |
| mPEG(**2000**)PLA(**1800**) /PLA-PFO | 1:9 | 1:5 | 120nm | 0.07 | Not measured | * |
| mPEG(**2000**)PLA(**1800**) /PLA-PFO | 2:8 | 1:1 | 210nm | 0.07 | 260nm | 0.1 |
| mPEG(**2000**)PLA(**1800**) /PLA-PFO | 2:8 | 1:3 | 236nm | 0.13 | 210nm | 0.1 |
| mPEG(**2000**)PLA(**1800**) /PLA-PFO | 2:8 | 1:5 | 325nm | 0.23 | 222nm | 0.4 |

### Ultrasound measurement

A focused sound field is established using a 1.0 MHz cavitation transducer (Panametrices V392) used at a pulse length of 32 cycles. The behavior of activated microcapsules is examined using a passive acoustic detector. The passive detector is composed of a broadband focused transducer (3.8 cm in diameter and 5.1 cm in focal length) with a center frequency of 5 MHz (Panametrics V307) and a broadband low-noise signal amplifier (40dB). A high-pass filter of 3.0 MHz (TTE HB5-3M-65B) and a low-pass filter of 10.7 MHz (MiniCircuits BLP-10.7) are employed to remove directly transmitted, diffraction-induced 1.0 MHz acoustic signals from the cavitation transducer. A digital oscilloscope (Model LT374L, LeCroy, Chestnut Ridge, NY) is used to digitize the amplified scattering signals with a sampling frequency of 20 MHz.

A time modulator (Four Channel Digital Delay/Pulse Generator; Standford Research Systems DG535) is used to synchronize the acoustic detector with the activation ultrasound pulses at PRF (pulse repetition frequency) of 2.0 Hz. The activation transducer is mounted horizontally on the sidewall of a rectangular test chamber (20.2x20.2x9.6 cm³) while the acoustic detector is placed vertically and aligned confocally at a right angle with the cavitation transducer. Because both transmit and receive transducers are focused transducers, the detector is very sensitive only to spheres in the small confocal region of the two transducers. With the passive technique, the activation threshold and post-activation oscillation (or activation-induced destruction) of microcapsules can be studied by characterizing the waveforms of received acoustic signals, and by analyzing harmonic and noise generation via the spectra of the signals. Activation event counts (or relative activation rates) of microcapsules for every 100 insonations of 1.0 MHz tonebursts were measured by automatically counting received scattered signals using LabView.

For acoustic activation measurements, each sample vial containing 1 ± 0.1 mg nanobubbles was reconstituted with 5 ml of deionized, air saturated water. An amount of 10 µL of this suspension was injected into a 3.4 L tank filled with air-saturated water that was left overnight to minimize the existence of minute gas pockets. While acoustic activation of nanobubbles was generated with the insonation of 1 MHz, 32-cycle ultrasound tonebursts, each acoustic event was detected according to the high harmonic content between 3 and 8 MHz of the scattered signals from the activated nanobubbles.

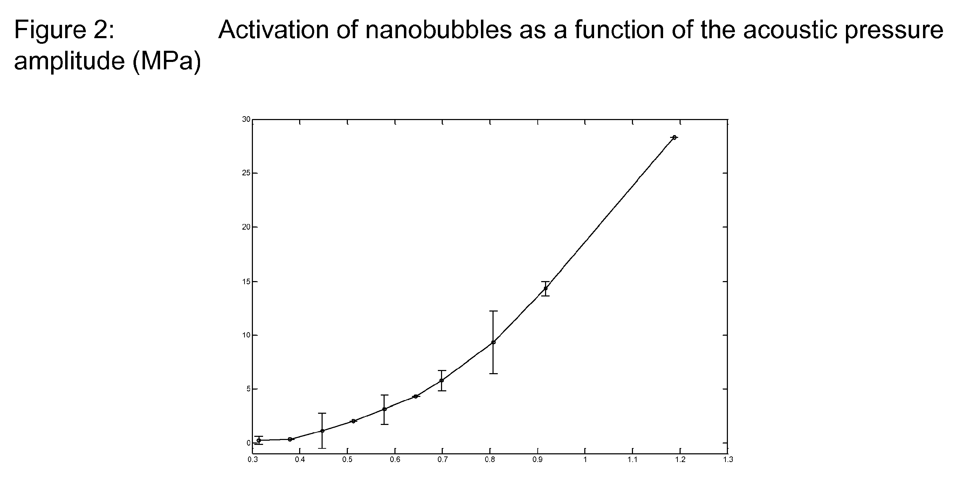

Figure 2 gives an example of an activation curve for sample mPEG(2000)PLA(1800)/PLA-PFO with a polymer to cyclodecane ration of 1:3 and pegylated to hydrophobically modified pla of 1:9. The acoustic event count is plotted as a function of the negative acoustic pressure amplitude in MPa at 1 MHz (or mechanical index), the other compositions show similar behaviour (to be added after re-measuring some of them). The negative acoustic pressure amplitude is directly responsible for the disintegration of nanobubbles and subsequent gas release from the disrupted nanobubbles (so-called "activation"). In addition, robust activation of such nanobubbles is observed (as bright flashes upon exposure to ultrasound pulses at an MI of 1.2) on an HDI-5000 ultrasound scanner with a P4-2 probe.

### In vivo experiments

Circulation time of conventional ultrasound contrast agents is generally below 30 minutes. To show prolonged circulation of spheres synthesized by the method according to the invention the following experiment was performed. Spheres loaded with the fluorescent dye nile red, were formed using a 1:1 mixture of cylco-octane to hexadecane to form the core from which the cyclo-octane fraction was removed by freeze-drying. A sample was reconstituted in saline and 100 µl was injected retro-orbitally into C57BL/6 mice, anaesthetized with isoflurane. Blood samples were taken and the animals were sacrificed after 4 and after 7 hours post injection. Liver, lungs, spleen, kidneys and heart were taken and weighted. Triton X100 (250 µl, 3% in water) was added followed by 750 µl water. The organs were ground using a sonicator (Sonicator Heat Systems W375 cell disruptor), freeze dried and the nile red was extracted using isopropanol. After centrifugation the fluorescence was measured (excitation 570 nm, detection 630 nm, cut off 590 nm). From these values and the sample (organ or blood) weights the relative amount of nile red was determined.

The distribution after 4 and after 7 hours was determined, showing predominant presence in liver and blood, each for 5 mice. The ratio of liver to blood is higher after 7 hours indicating that slow liver accumulation takes place, however, even after 7 hours a significant fraction is still present in the circulation.

## Claims

1. A method for synthesis of a hollow sphere with a polymeric shell comprising the following steps:
(a) mixing a solution comprising a polymer (1) with a hydrophilic block, a hydrophobic compound, a water miscible organic solvent with an aqueous solution
(b) removal of water miscible organic solvent
(c) lyophilizing to remove hydrophobic alkane

2. A method according to claim 1, wherein the hydrophilic organic solvent is selected from the group comprising acetone, tetrahydrofuran, dimethylsulfoxide, dimethylformamide, dimethylacetamide or any combination thereof.

3. A method according to any of claim 1 to 2, wherein the polymer is (1) is selected from the group comprising polylactide, polycaprolactone, polycyanoacrylate and copolymers of one of the foregoing, copolymers of polyglycolide, or any combination thereof.

4. A method according to claim 1 to 3 wherein an additional polymer (2) is added comprising an alkyl or a fluorinated end group.

5. A method according to anyone of claims 1 to 4 wherein the mixture comprises a hydrophobic compound that remains after step (c) and a pharmaceutical or diagnostic compound.

6. A method according to claim 5 wherein the remaining hydrophobic compound is selected from the group comprising alkanes with at least 16 carbon atoms, lipids, paraffin or oils.

7. A hollow sphere with a polymeric shell synthesized according to anyone of the foregoing claims.

8. A hollow sphere according to claim 7 with a size below 1 micron.

9. A hollow sphere according to any one of claims 7 and 8 that is able to release the gaseous contents upon application of ultrasound.

10. A hollow sphere according to any of claim 7 to 9 wherein the hollow sphere contains a gaseous pre-cursor.

11. A hollow sphere according to any of claim 7 to 10 wherein the polymeric shell of the sphere includes a targeting moiety.

12. A hollow sphere according to any of claim 7 to 11 wherein the sphere comprises a pharmaceutically active molecule.

13. A hollow sphere according to any one of claims 7 to 12 comprising an imaging agent selected from the group consisting of PET, SPECT or MRI agents

14. Use of hollow spheres obtained by the method of anyone of claims 1 to 6 or as defined in claim 7 to 13 in ultrasound imaging or ultrasound triggered drug delivery.
